# EUROPÄISCHE PATENTSCHRIFT

(11) **EP 0 959 876 B1**
(45) Veröffentlichungstag und Bekanntmachung des Hinweises auf die Patenterteilung: **07.05.2003**
(21) Anmeldenummer: 98908042.9
(22) Anmeldetag: 03.02.1998
(51) Int. Cl.: A61K 9/20

(54) **VERFAHREN ZUR TRENNUNG VON WIRKSTOFFEN IN FESTEN PHARMAZEUTISCHEN ZUBEREITUNGEN**
METHOD FOR SEPARATING ACTIVE SUBSTANCES IN SOLID PHARMACEUTICAL PREPARATIONS
PROCEDE POUR SEPARER DES PRINCIPES ACTIFS DANS DES PREPARATIONS PHARMACEUTIQUES SOLIDES

(30) Priorität: 14.02.1997 DE 19705538
(43) Veröffentlichungstag der Anmeldung: 01.12.1999
(73) Patentinhaber: GÖDECKE AKTIENGESELLSCHAFT, D-10587 Berlin (DE)
(72) Erfinder: WEILAND-WAIBEL, Andrea, 85662 Hohenbrunn (DE); RAUCHFUSS, Roland, D-79114 Freiburg (DE); BAMBACH, Thomas, D-79418 Schliengen (DE); SEEGMÜLLER, Lothar, D-78185 Karlsruhe (DE); SCHRIMPL, Leopold, D-79312 Emmendingen (DE)
(74) Vertreter: Mansmann, Ivo
(86) Internationale Anmeldenummer: EP9800557
(87) Internationale Veröffentlichungsnummer: WO98035655

(56) Entgegenhaltungen:
- EP-A- 0 566 393
- WO-A-93/24110
- DE-A- 1 948 019
- GB-A- 1 443 923

## Beschreibung

Aus der EP 0043254 ist ein Verfahren zur Herstellung einer Arzneimittelzubereitung mit verzögerter Wirkstoffabgabe bekannt, das dadurch gekennzeichnet ist, daß man (a) den Wirkstoff fein verteilt; (b) den Wirkstoff in feinverteilter Form sowohl mit einer feinverteilten hochschmelzenden lipiden oder lipoiden Komponente als auch mit einer fein verteilten niedrigschmelzenden lipiden oder lipoiden Komponente vermischt, wobei das Gewichtsverhältnis der beiden lipiden oder lipoiden Komponenten im Bereich 1:5 bis 5:1 liegt; (c) das resultierende Gemisch aus Wirkstoff und lipiden oder lipoiden Komponenten auf eine Temperatur bringt, die oberhalb des Schmelzpunkts der niedrigschmelzenden Komponente jedoch unterhalb des Schmelzpunkts der hochschmelzenden Komponente liegt, wobei der Wirkstoff und die hochschmelzende lipide oder lipoide Komponente gleichmäßig in der vollständig geschmolzenen niedrigschmelzenden lipiden oder lipoiden Komponente dispergiert werden; (d) das resultierende Gemisch nach dem Aufschmelzen der niedrigschmelzenden Komponente unter deren Schmelzpunkt abkühlen läßt und (e) das resultierende Gemisch während des Abkühlens oder danach granuliert, wobei die Angaben "niedrigschmelzend" und "hochschmelzend" in Bezug auf ihre Relation zueinander verwendet werden, ohne irgendwelche besondere Schmelzpunkte einzuschließen.

Das Verfahren gestattet die Herstellung von Retardformulierungen bei relativ niedriger Temperatur und dient vor allem der Schonung hitzeempfindlicher Wirkstoffe.

In Weiterführung der Entwicklung des Verfahrens gemäß EP 0043254 beschreibt die EP 0641195 ein Extrusionsverfahren bei dem man die gut zerkleinerte und vorgemischte pulverförmige Masse gemäß (b) mittels einer Extruderschnecke in einen vortemperierten Extruder einführt und so gemäß (c) auf eine Temperatur bringt, die höchstens 4°C über der Schmelztemperatur der niedrigschmelzenden lipiden oder lipoiden Komponente liegt, das so erwärmte Gemisch bei einem Druck von 200 bis 600 kPa(N/m²) einem Extrusionsprozess unterwirft und es auf diese Weise dispergiert, wobei die teilaufgeschmolzene und gut vermischte Masse durch eine Düsenplatte mit einem Düsendurchmesser von 1,2 bis 4 mm extrudiert und anschließend gemäß (d) abgekühlt und gewünschtenfalls anschließend granuliert wird.

Die beschriebenen Verfahren befassen sich mit Retardzubereitungen, die nur einen Wirkstoff enthalten. Da der Vorteil des beschriebenen Standes der Technik gerade darauf beruht, als eigentlichen Wirkstoffträger ausschließlich die niedrigerschmelzende lipoide Komponente einzusetzen, enthalten beide Dokumente immanent die Anweisung an den Fachmann, beim Einsatz von zwei oder mehr Wirkstoffen ebenfalls nur von einer lipoiden Komponente Gebrauch zu machen und die andere Komponente als Ballastkomponente zu betrachten, die wirkstofffrei bleiben soll.

Die GB 144 392 offenbart ein Verfahren zur Herstellung einer Arzneimittelzubereitung mit verzögerter Wirkstoffabgabe, in dem z.B. entgegenwirkende Wirkstoffe mit einer Fettsäure ummantelt werden, wobei die jeweiligen Fettsäure unterschiedliche Schmelzpunkte aufweisen.

In der Praxis ergibt es sich bisweilen, daß beim Einsatz von mehr als einem Wirkstoff in Kombinationspräparaten Unverträglichkeiten der Wirkstoffe untereinander auftreten.

Bisweilen benötigt auch einer der Wirkstoffe besondere Hilfsstoffe wie z.B Antioxidantien.

Es wurde nun gefunden, daß sich speziell die obenbeschriebenen-Retardprodukte hervorragend dazu eignen, unverträgliche Wirkstoffe auf äußerst elegante Weise räumlich voneinander zu trennen und so die Stabilität von festen Arzneimitteln wie z.B. Tabletten oder Granulaten erheblich zu erhöhen, wobei sich die Trennmaßnahme technisch besonders vorteilhaft und kostengünstig gestalten läßt.

Aufgabe der Erfindung ist es, die Stabilität von festen Arzneimittelformulierungen wie z.B. Tabletten, Dragees oder Granulaten insbesondere bei Unverträglichkeiten der Wirkstoffe untereinander oder bei Unverträglichkeiten mit bestimmten Hilfsstoffen durch eine räumliche Trennung der unvertraglichen Komponenten herbeizuführen.

Die Aufgabe wurde dadurch gelöst, daß man bei Arzneimittelformulierungen gemäß EP 043254 den jeweils temperaturunempfindlicheren Wirkstoff und/oder Hilfsstoff in die höherschmelzende lipide oder lipoide Komponente einarbeitet und diese in fester und feinverteilter Form mit der niedrigerschmelzenden lipiden oder lipoiden Komponente und dem zweiten temperaturempfindlicheren Wirkstoff und/oder Hilfsstoff gemäß EP 0 043 254 oder EP 0 641 195 zu Arzneimitteln weiterverarbeitet. Aus der EP 0 452 145 sind zwar weitere oral verabreichbare beschichtete Zusammensetzungen bekannt, bei denen Körner eines hitzebeständigen pharmazeutischen Wirkstoffs durch geringe Mengen eines ersten thermoschmelzenden Materials so gebunden werden, daß sie aneinander haften und dann von einem zweiten thermoschmelzenden Material, dessen Schmelzpunkt tiefer als der des ersten liegt, beschichtet werden. Hierbei entstehen Granulen eines einzelnen Wirkstoffs, die jeweils mit lipidem Material beschichtet sind. Es ist jedoch ersichtlich, daß dieses Prinzip nicht zu einer Lösung der erfindungsgemäßen Aufgabe beitragen kann, da ein zweiter Wirkstoff oder unverträglicher Hilfsstoff weder vorgesehen ist, noch gegebenenfalls getrennt vom ersten Wirkstoff in die Zusammensetzung eingebracht werden kann.

Da beim erfindungsgemäßen Aufschmelzen der niedrigerschmelzenden Komponente die höherschmelzende Komponente unverändert im festen Aggregatzustand verbleibt, sind die Wirkstoffteilchen der höherschmelzenden Komponente auch beim Aufschmelzen der niedrigerschmelzenden lipiden oder lipoiden Komponente vollständig und dauernd in eine feste lipide oder lipoide Phase eingebettet, so daß Kontakte mit den unverträglichen Anteilen aus der niedrigerschmelzenden Komponente sicher verhindert werden.

Die Retardierung läßt sich durch Zugabe hydrophiler Bestandteile in weiten Grenzen regeln, so daß auch nichtretardierte Formen nach dem Verfahren gemäß vorliegender Erfindung hergestellt werden können. Besonders elegant läßt sich der Retardierungsgrad mittels hydrophiler Polymerer gemäß EP 0 068 446 einstellen.

Somit ermöglicht die Erfindung die Herstellung einfach und kostengünstig herzustellender fester Arzneimittelformen mit beliebig einzustellender Wirkstofffreigabe, insbesondere wenn mindestens ein Wirkstoff entweder gegenüber einem weiteren Wirkstoff oder einem Hilfsstoff unverträglich ist und geschützt werden soll. Dadurch daß die Wirkstoffe durch die höherschmelzende Komponente praktisch vollständig maskiert werden können und im weiteren Verfahren und später bei der Aufbewahrung bis zum Verbrauch des fertigen Arzneimittels diese Maskierung erhalten bleibt, können auch mehrere hochschmelzende Komponenten mit unterschiedlichen Wirkstoffen oder unterschiedlichem Wirkstoffgehalt eingesetzt werden, die dann mit der niedrigerschmelzenden Komponente, in die besonders temperaturempfindliche Wirkstoffe eingearbeitet werden können, zum fertigen Arzneimittel verbunden werden.

Gegenstand der vorliegenden Erfindung ist somit ein Verfahren zur räumlichen Trennung von mindestens zwei unverträglichen Wirkstoffen (bzw. Hilfsstoffen) A und B in festen Arzneimittelformen bei dem man
a) die Wirkstoffe A und B fein verteilt;
b) den Wirkstoff B in feinverteilter Form sowohl mit einer feinverteilten hochschmelzenden lipiden oder lipoiden Komponente als auch mit einer fein verteilten niedrigschmelzenden lipiden oder lipoiden Komponente vermischt, wobei das Gewichtsverhältnis der beiden lipiden oder lipoiden Komponenten im Bereich 1:5 bis 5:1 liegt;
c) das resultierende Gemisch aus Wirkstoff B und lipiden oder lipoiden Komponenten auf eine Temperatur bringt, die oberhalb des Schmelzpunkts der niedrigschmelzenden Komponente jedoch unterhalb des Schmelzpunkts der hochschmelzenden Komponente liegt, wobei der Wirkstoff und die hochschmelzende lipide oder lipoide Komponente gleichmäßig in der vollständig geschmolzenen niedrigschmelzenden lipiden oder lipoiden Komponente dispergiert werden;
d) das resultierende Gemisch nach dem Aufschmelzen der niedrigschmelzenden Komponente unter deren Schmelzpunkt abkühlen läßt und
e) das resultierende Gemisch während des Abkühlens oder danach granuliert, wobei die Angaben
   "niedrigschmelzend" und "hochschmelzend" in Bezug auf ihre Relation zueinander verwendet werden, ohne irgendwelche besondere Schmelzpunkte einzuschließen,
   das dadurch gekennzeichnet ist,
daß man vor dem Schritt (b) den feinverteilten Wirkstoff A gleichmäßig in die höherschmelzende lipide oder lipoide Komponente einarbeitet und das so erhaltene

Gemisch granuliert oder auf andere Weise fein verteilt. Die besonderen Ausführungsformen der vorliegenden Erfindung werden die den abhängigen Ansprüchen angegeben.

Beim Einsatz besonders oxidationsempfindlicher Wirkstoffe, kann der Schutz der hochschmelzenden lipiden oder lipoiden Komponente gegen oxidative Einflüsse, dadurch gesteigert werden, daß man den getrennten Komponenten wahlweise spezielle Antioxidantien zusetzt. Diese können hydrophiler oder lipophiler Art sein. Zu den hydrophilen Antioxidantien gehört beispielsweise Ascorbinsäure. Lipophile Antioxidantien umfassen Ascorbinsäureester, BHA, BHT, Gallussäureester, Tocopherole, und oder Nordihydroguajaretsäure. Weitere Hilfsstoffe dieser Art sind Alkali- oder Erdalkalisulfite oder-bisulfite in einer Menge von 0,001 bis 5 Gew%, bevorzugt 0,001 bis 1%, bezogen auf die Gesamtmasse der entsprechenden Komponente. Deren Wirkung kann durch Synergisten wie z.B. Ascorbinsäure, Citronensäure und deren Salze, Lecithin oder Ethylendiamin-tetraessigsäure verstärkt werden.

Die Einarbeitung des Wirkstoffs A in die höherschmelzende Komponente kann durch einfaches Aufschmelzen und Granulation der abgekühlten Masse erfolgen. Es ist jedoch auch ein Überzug des Wirkstoffs im Wirbelschichtverfahren (bottom-, top-, oder tangential spray) möglich. Dabei entsteht ein feines Granulat, daß unmittelbar weiterverarbeitet werden kann. Der Wirkstoff kann ferner gleichmäßig in der geschmolzenen Komponente verteilt und in einem Sprühturm abgekühlt werden (Spray congealing).

Bezüglich der Weiterverarbeitung wird auf die Verfahren des eingangs genannten Standes der Technik verwiesen.

### Beispiel 1

### Tabletten enthaltend 500 mg Acetylsalicylsäure und 60 mg Phenylephrine

| Zusammensetzung | |
|---|---|
| ASS | 500 mg/Tablette |
| Phenylephrine | 60 mg/Tablette |
| Lactose | 256 mg/Tablette |
| Rizinusöl, hydr. | 40 mg/Tablette |
| Stearinsäure | 60 mg/Tablette |
| Magnesiumstearat | 2,0 mg/Tablette |
| Carboxymethylcell.-Na | 2,0 mg/Tablette |

30 kg Phenylephrine werden mit 20 kg hydrierten Rizinusöls partikelgecoatet. Die erhaltenen Partikel werden dann in einer Vormischung mit 250 kg Acetylsalicylsäure, 128 kg Lactose und 30 kg Stearinsäure über eine Dosierschnecke einem Extruder zugeleitet. Der Extruder ist in mehrere getrennt temperierbare Schüsse eingeteilt. Die Mischung wird bei einer Umdrehungszahl von 170 - 180 UpM in den durch den heizbaren Mantel vorgeheizten Extruder gefördert. Der Schneckendurchmesser beträgt 50 mm. Die Manteltemperatur beträgt in allen Schüssen 58 - 60°C. Nach einer durchschnittlichen Verweildauer von 2 - 4 Minuten wird das teilaufgeschmolzene Produkt durch eine Düsenplatte extrudiert. Die Düsenplatte enthält 20 Düsenöffnungen mit einem inneren Durchmesser von 1,5 - 3 mm. Die Apparatur fördert unter den beschriebenen Bedingungen 110 - 120 kg Extrudat in der Stunde. Das Extrudat wird in Form feiner gleichmäßiger Stränge auf dem langsam laufenden Förderband weitgehend auf Raumtemperatur abgekühlt und schließlich einem Granulator zugeführt. Das fertige Granulat gelangt schließlich in einen Auffangbehälter. Anschließend wird das Granulat mit 1 kg Magnesiumstearat und 1 kg Natriumcarboxymethylcellulose (Außenphase) gleichmäßig vermischt und in üblicher Weise zu Tabletten mit einem Gesamtgewicht von 920 mg verpreßt.

### Beispiel 2

### Tabletten enthaltend Ascorbinsäure und Vitamin B12 (Cyanocobalamin)

In der in Beispiel 1 beschriebenen Weise werden 500 kg Ascorbinsäure, 0,5 kg Vitamin B12, 50 kg Lactose, 20 kg Rizinusöl hydriert, 27,5 kg Glycerinmonostearat, 1 kg Hydroxyethylcellulose und 1 kg Magnesiumstearat zu Tabletten verarbeitet. Vor dem Extrusionsprozeß wird das Vitamin B12 (Cyanocabalamin) mit dem hydrierten Rizinusöl partikelgecoatet. Man erhält Tabletten mit einem Gesamtgewicht von 1200 mg und einem Gehalt von je 1000 mg Ascorbinsäure und 1 mg Vitamin B12,

| | |
|---|---|
| Ascorbinsäure | 1000 mg/Tablette |
| Cyanocobalamin | 1 mg/Tablette |
| Lactose | 100 mg/Tablette |
| Rizinusöl, hydriert | 40 mg/Tablette |
| Glycerinmonostearat | 55 mg/Tablette |
| Hydroxyethylcellulose | 2 mg/Tablette |
| Magnesiumstearat | 2 mg/Tablette |
| Summe | 1200 mg/Tablette |

## Patentansprüche

1. Verfahren zur räumlichen Trennung von mindestens zwei Wirkstoffen bzw. Hilfsstoffen A und B in festen Arzneimittelformen, bei dem man
a) die Wirkstoffe A und B fein verteilt;
b) den Wirkstoff B in feinverteilter Form sowohl mit einer feinverteilten hochschmelzenden lipiden oder lipoiden Komponente als auch mit einer fein verteilten niedrigschmelzenden lipiden oder lipoiden Komponente vermischt, wobei das Gewichtsverhältnis der beiden lipiden oder lipoiden Komponenten im Bereich 1:5 bis 5:1 liegt;
c) das resultierende Gemisch aus Wirkstoff B und lipiden oder lipoiden Komponenten auf eine Temperatur bringt, die oberhalb des Schmelzpunkts der niedrigschmelzenden Komponente jedoch unterhalb des Schmelzpunkts der hochschmelzenden Komponente liegt, wobei der Wirkstoff und die hochschmelzende lipide oder lipoide Komponente gleichmäßig in der vollständig geschmolzenen niedrigschmelzenden lipiden oder lipoiden Komponente dispergiert werden;
d) das resultierende Gemisch nach dem Aufschmelzen der niedrigschmelzenden Komponente unter deren Schmelzpunkt abkühlen läßt und
e) das resultierende Gemisch während des Abkühlens oder danach granuliert, wobei die Angaben "niedrigschmelzend" und "hochschmelzend" in Bezug auf ihre Relation zueinander verwendet werden, ohne irgendwelche besonderen Schmelzpunkte einzuschließen,
**dadurch gekennzeichnet,**
**daß** man vor dem Schritt (b) den feinverteilten Wirkstoff A gleichmäßig in die höherschmelzende lipide oder lipoide Komponente einarbeitet und das so erhaltene Gemisch granuliert oder auf andere Weise fein verteilt.

2. Verfahren nach Anspruch 1, **dadurch gekennzeichnet, daß** man mindestens einer der lipiden oder lipoiden Komponente ein Antioxidationsmittel zusetzt.

3. Verfahren nach Anspruch 2, **dadurch gekennzeichnet, daß** das Antioxidationsmittel Ascorbinsäure, Ascorbinsäureester, BHA, BHT, Gallussäureester, Tocopherole, Nordihydroguajaretsäure, Alkali- oder Erdalkalisulfite oder Bisulfite umfaßt.

4. Verfahren gemäß Anspruch 1 bis 3, **dadurch gekennzeichnet, daß** als höherschmelzende lipide oder lipoide Komponente hydriertes Pflanzenöl, insbesondere hydriertes Rizinusöl verwendet wird.

5. Verfahren gemäß Anspruch 1 bis 4, **dadurch gekennzeichnet, daß** als niedrigerschmelzende Komponente eine höhere Fettsäure oder ein Mono- Di- oder Triester einer höheren Fettsäure mit einem Schmelzpunkt von 50-60°C verwendet wird.

6. Verfahren nach Anspruch 5, **dadurch gekennzeichnet, daß** als höhere Fettsäure Palmitin- und/oder Stearinsäure oder Glycerin-Behenat verwendet wird.

## Claims

1. Process for spatial separation of at least two active ingredients or auxiliaries A and B in solid medicament forms, in which
a) the active ingredients A and B are finely divided;
b) the active ingredient B in finely divided form is mixed both with a finely divided high-melting lipid or lipoid component and with a finely divided low-melting lipid or lipoid component, wherein the weight ratio of the two lipid or lipoid components lies in the range 1:5 to 5:1;
c) the resulting mixture of active ingredient B and lipid or lipoid components is brought to a temperature which lies above the melting point of the low-melting component but below the melting point of the high-melting component, wherein the active ingredient and the high-melting lipid or lipoid component are dispersed uniformly in the completely melted low-melting lipid or lipoid component;
d) after melting the low-melting component, the resulting mixture is allowed to cool below the melting point thereof and
e) the resulting mixture is granulated during cooling or thereafter, wherein the statements "low-melting" and "high-melting" are used with reference to their relation to one another without including any particular melting points,
**characterised in that** before step (b), the finely divided active ingredient A is incorporated uniformly into the higher-melting lipid or lipoid component and the mixture thus obtained is granulated or finely divided in a different manner.

2. Process according to claim 1, **characterised in that** an anti-oxidant is added to at least one of the lipid or lipoid component.

3. Process according to claim 2, **characterised in that** the anti-oxidant includes ascorbic acid, ascorbates, BHA, BHT, gallates, tocopherols, nordihydroguaiaretic acid, alkali metal or alkaline earth metal sulphites or bisulphites.

4. Process according to claim 1 to 3, **characterised in that** hydrogenated vegetable oil, in particular hydrogenated castor oil, is used as higher-melting lipid or lipoid component.

5. Process according to claim 1 to 4, **characterised in that** a higher fatty acid or a mono-, di- or triester of a higher fatty acid having a melting point of 50-60°C is used as lower-melting component.

6. Process according to claim 5, **characterised in that** palmitic and/or stearic acid or glycerol behenate is used as higher fatty acid.

## Revendications

1. Procédé pour séparer dans l'espace au moins deux substances actives ou substances auxiliaires A et B dans des formes solides de médicaments, dans lequel
a) on divise finement les substances actives A et B ;
b) on mélange la substance active B sous la forme finement divisée à la fois avec un composant lipidique ou lipoïdique de haut point de fusion finement divisé et avec un composant lipidique ou lipoïdique de bas point de fusion finement divisé, le rapport de mélange des deux composants lipidiques ou lipoïdiques se situant dans la plage de 1:5 à 5:1 ;
c) on porte le mélange résultant de substance active B et de composants lipidiques ou lipoïdiques à une température qui est supérieure au point du fusion du composant de bas point de fusion mais inférieure au point de fusion du composant de haut point de fusion, ce qui fait que la substance active et le composant lipidique ou lipoïdique de haut point de fusion se dispersent uniformément dans le composant lipidique ou lipoïdique de bas point de fusion totalement fondu ;
d) on laisse refroidir le mélange résultant, après la fusion du composant de bas point de fusion, au-dessous du point de fusion de ce dernier et
e) on granule le mélange résultant pendant ou après le refroidissement, les notions de "bas point de fusion" et "haut point de fusion" étant utilisées à titre comparatif sans comporter aucun point de fusion particulier,
procédé **caractérisé en ce qu'**on incorpore uniformément avant l'étape (b) la substance active A finement divisée au composant lipidique ou lipoïdique de plus haut point de fusion et on granule ou on divise finement d'une autre façon le mélange ainsi obtenu.

2. Procédé suivant la revendication 1, **caractérisé en ce qu'**on ajoute un agent antioxydant à l'un au moins des composants lipidiques ou lipoïdiques.

3. Procédé suivant la revendication 2, **caractérisé en ce que** l'agent antioxydant comprend l'acide ascorbique, un ester d'acide ascorbique, le BHA, le BHT, un ester d'acide gallique, des tocophérols, l'acide nordihydroguaiarétique, des sulfites ou bisulfites de métaux alcalins ou alcalino-terreux.

4. Procédé suivant les revendications 1 à 3, **caractérisé en ce qu'**on utilise comme composant lipidique ou lipoïdique de plus haut point de fusion une huile végétale hydrogénée, en particulier l'huile de ricin hydrogénée.

5. Procédé suivant les revendications 1 à 4, **caractérisé en ce qu'**on utilise comme composant de plus bas point de fusion un acide gras supérieur ou un monoester, diester ou triester d'un acide gras supérieur ayant un point de fusion de 50 à 60°C.

6. Procédé suivant la revendication 5, **caractérisé en ce qu'**on utilise comme acide gras supérieur l'acide palmitique et/ou l'acide stéarique ou le béhénate de glycérol.
